# EUROPEAN PATENT SPECIFICATION

(11) **EP 1 454 618 B1**
(45) Date of publication and mention of the grant of the patent: **02.03.2016**
(21) Application number: 04004834.0
(22) Date of filing: 02.03.2004
(51) Int. Cl.: A61Q 11/00, A61K 8/64, A61K 8/02, A61K 31/695

(54) **Anticaries compositions containing phaseolamin**
Antikariesmittel Phaseolamin enthaltend
Composition anti-carie comprenant la Phaseolamine

(30) Priority: 04.03.2003 IT MI20030391
(43) Date of publication of application: 08.09.2004
(73) Proprietor: S.I.I.T. S.r.l. Servizio Internazionale Imballaggi Termosaldanti, 20090 Trezzano S/N (Milan) (IT)
(72) Inventor: Di Pierro, Francesco, 20090 Trezzano S/N (Milan) (IT)
(74) Representative: Minoja, Fabrizio

(56) References cited:
- EP-A- 0 179 324
- EP-A- 1 295 535
- WO-A-01/17369
- WO-A-02/094221
- US-A- 4 217 341
- PATENT ABSTRACTS OF JAPAN vol. 0163, no. 83 (C-0974), 17 August 1992 (1992-08-17) & JP 4 124124 A (MORISHITA JINTAN KK), 24 April 1992 (1992-04-24)
- DATABASE WPI Section Ch, Week 199338 Derwent Publications Ltd., London, GB; Class B07, AN 1993-300605 XP002283997 & RO 103 923 A (NIVEA INTR PROD COSMETICE) 25 August 1992 (1992-08-25)

## Description

### FIELD OF THE INVENTION

The present invention relates to anticaries compositions containing salivar ptyalin inhibitors.

More particularly, the present invention relates to anticaries compositions containing phaseolamin.

### STATE OF THE ART

Dental caries is a destructive process that affects the hard tissues of the tooth. Although its main cause are the acids produced by the bacteria of the dental plaque, other concomitant factors, such as host susceptibility and presence of sugars and cariogenic bacteria, contribute to its development.

As far as host susceptibility is concerned, tooth structure plays a remarkable role in caries onset. For example, food residues and bacteria tend to settle on teeth carved with deep fissures. Plaque also accumulates on crowded teeth, as narrow interdental spaces cannot be easily reached by the toothbrush. Moreover, some physiological conditions, such as hormonal changes occurring during pregnacy and milking, make the saliva more viscous, thus increasing bacterial adhesiveness to the tooth and susceptibility to caries.

Sugars are also a major determinant: they are metabolised to acids by certain bacteria usually present in the oral cavity and such acids cause tooth demineralisation and start tooth decay. Caries is caused not only by simple sugars, primarily glucose and saccharose, but also by complex sugars, mainly starch contained in pasta and bread. Starch is a glucose polymer digested in the intestine by pancreatic α-amylase; glucose is subsequently absorbed and the glycemic level increases. Actually, starch digestion does not start in the intestine, but in the oral cavity, through the action of ptyalin, a salivar α-amylase. Ptyalin provides on the tooth surface a considerable amount of simple sugars that are metabolised by the bacteria of the dental plaque into acids responsible for demineralisation. In other words, starch residues (pasta, bread, potatoes and the like) are indirectly responsible for caries onset.

Among the microbial species found in the mouth (more than 350), the main causes of caries lesions are *streptococcus mutans* and *lactobacilli;* the former, in particular, sticks to the enamel surface and builds up a polysaccharides net to which a number of other microrganisms adhere, thus causing the formation of the so-called "bacterial plaque".

A good oral hygiene, through brushing and flossing, is necessary to prevent caries. To remove cariogenic substances and plaque from the oral cavity, teeth should be thoroughly brushed after each meal, in particular before bedtime, because at night food residues are easily metabolised to acids. Nevertheless, good oral hygiene might not be sufficient in the case of individuals having a particular predisposition to caries. Moreover, good oral hygiene (brushing and flossing) cannot be easily maintained during working hours.

Several attempts have been made for some years to reduce caries incidence, but an effective remedy has not been found to date. Fluorine proved particularly effective: daily assumption of fluorine tablets or drops during tooth mineralization (a process that terminates at the age of 12) makes the enamel more resistant to acids; when mineralisation is complete, systemically-administered fluorine is not able do reach the enamel. On the contrary, topical use of fluorine is effective at any age, as it both reinforces the enamel and inhibits acids production. Fluorine (optionally in combination with chlorhexidine) has also been used for some time in mouthwashes or medicated toothpastes, but their beneficial effects are limited.

To solve these problems, lacquers that provide targeted, sustained release of small amounts of fluorine or chlorhexidine have been recently put on the market. Thus, susceptible subjects can be treated with products that avoid caries formation, such as sealers and lacquers based on fluorine or chlorhexidine. Sealers are resins which are applied on the chewing surfaces of healthy teeth in order to reduce the depth of the fissures wherein caries originates. Nevertheless, sealers are sparingly used to date, as they cause a sensation of discomfort.

A further, widespread caries remedy are alkaline substances (such as bicarbonates), that are able to buffer excessive acidity of the oral cavity.

It will be appreciated that none of the aforementioned remedies counteracts ptyalin action, i.e. metabolisation of starch to highly cariogenic simple sugars. Starch residues, being sticky in nature, are nowadays deemed to be more dangerous than simple sugars that are soluble and easily eluted away from the oral cavity.

JP 4124124 discloses a formulation comprising powder of beans of several species for deodorizing effects of foul breath and unpleasant garlic smell.

WO 01/17369 and WO 02/094221 disclose compositions comprising phaseolamin for reducing absorption of sugars and for weight control.

EP 1295535, published after the present priority date, discloses compositions of phaseolamin for controlling carbohydrate cravings and inducing weight loss.

### DESCRIPTION OF THE INVENTION

The present invention relates to compositions containing salivar ptyalin inhibitors, in particular phaseolamin.

Phaseolamin, extracted from common bean (*Phaseolus vulgaris),* is a thermolabile and gastrolabile protein having anti-ptyalin activity, that specifically inhibits conversion of starch to simple sugars. This inhibition makes starch residues harmless and stops from the beginning the metabolisation of sugars to acids, in particular lactic acid, which cause tooth demineralisation and caries.

The compositions of the present invention can be in the solid form, for example mouth-soluble, slow-release tablets, candies or chewing gums; in the semi-solid form, such as gels, toothpastes, and the like; or in the liquid form, such as mouth-washes and the like.

The compositions of the present invention can optionally comprise auxiliary substances, such as alkali (alkali carbonates or bicarbonates), inorganic fluorides, such as zinc fluoride, sodium fluoride, potassium fluoride, chlorhexidine or derivatives thereof or other bacteriostatic and bactericidal compounds, anti-inflammatory and soothing agents such as 18β-glycyrrhizinic acid, *Krameria triandra* extracts, green tea, etc..

Particularly preferred are compositions containing, in addition to phaseolamin, one or more of alkali carbonates or bicarbonates, sodium monofluorophosphate, inorganic fluorides, chlorhexidine and derivatives thereof, 18β-glycyrrhizinic acid, *Krameria triandra* extract, green tea extract.

The compositions of the invention can be prepared according to conventional techniques, and can contain excipients selected from thickening agents, such as base gum (in the case of chewing gums), hydroxypropyl cellulose, hydroxyethylcellulose, guar gum, gum arabic, cellulose gum; wetting agents; sweetening agents, in particular non cariogenic sweetening agents, such as xylitol, maltitol, acesulfame K, maltitol, aspartame; flavours; whitenings; abrasives, such as phosphate derivatives, preferably sodium monofluorophosphate, tricalcium phosphate, zinc orthophosphate, hydrate alumina, calcium carbonate, bentonite; desensitizing agents, such as potassium and strontium salts; zinc citrate; triclosan.

The weight amount of phaseolamin in the compositions of the invention can vary within broad ranges, but is typically from about 0.01 to about 2%, preferably from about 0.1 to about 1%.

The concentrations of the other ingredients are in principle similar to those of similar products for oral and dental hygiene.

The following examples illustrate the invention in greater detail.

**EXAMPLE 1**

| **SLOW-RELEASE ANTICARIES COMPOSITION 1** | | |
|---|---|---|
| **Type: 500 mg tablets** | | |
| **Daily dosage: 1 tablet after each meal** | | |
| **INGREDIENT** | **AMOUNT mg** | **%** |
| Sorbitol | 447.790 | 89.56 |
| Flavour | 30.000 | 6.00 |
| **Phaseolamin** | 5.000 | 1.00 |
| Aspartame | 10.000 | 2.00 |
| **Sodium Fluoride** | 2.210 | 0.44 |
| Magnesium stearate | 5.000 | 1.00 |
| | | |
| **TOTAL** | **500** | |

**EXAMPLE 2**

| **SLOW-RELEASE ANTICARIES COMPOSITION 2** | | |
|---|---|---|
| **Type: 500 mg tablets** | | |
| **Daily dosage: 1 tablet after each meal** | | |
| **INGREDIENT** | **AMOUNT mg** | **%** |
| Saccharose | 455.000 | 91.00 |
| Flavour | 30.000 | 6.00 |
| **Phaseolamin** | 5.000 | 1.00 |
| 18β-glycyrrhizinic acid | 5.000 | 1.00 |
| Magnesium stearate | 5.000 | 1.00 |
| | | |
| **TOTAL** | **500** | |

**EXAMPLE 3**

| **1.35 g ANTICARIES CHEWING GUM 1** | | |
|---|---|---|
| **Daily dosage: 1 dose after each meal** | | |
| **INGREDIENT** | **AMOUNT mg** | **%** |
| Sorbitol | 600.000 | 44.44 |
| Isomalt | 300.000 | 22.22 |
| Xylitol | 200.000 | 14.81 |
| Maltitol syrup | 89.500 | 6.63 |
| Gum base | 70.000 | 5.19 |
| Aspartame | 12.000 | 0.89 |
| Acesulfame K | 12.000 | 0.89 |
| Flavour | 12.000 | 0.89 |
| Titanium dioxide E171 | 12.000 | 0.89 |
| Gum arabic | 15.000 | 1.11 |
| **18β-glycyrrhizinic acid** | 10.000 | 0.74 |
| **Sodium bicarbonate** | 5.500 | 0.41 |
| **Phaseolamin** | 2.000 | 0.15 |
| Carnauba wax | 5.000 | 0.37 |
| E320 | 5.000 | 0.37 |
| | | |
| **TOTAL** | **1.350** | |

**EXAMPLE 4**

| **1.35 g ANTICARIES CHEWING GUM 2** | | |
|---|---|---|
| **Daily dosage: 1 dose after each meal** | | |
| **INGREDIENT** | **AMOUNT mg** | **%** |
| Sorbitol | 600.000 | 44.44 |
| Isomalt | 300.000 | 22.22 |
| Xylitol | 200.000 | 14.81 |
| Maltitol syrup | 98.395 | 7.29 |
| Gum base | 70.000 | 5.19 |
| Aspartame | 12.000 | 0.89 |
| Acesulfame K | 12.000 | 0.89 |
| Flavour | 12.000 | 0.89 |
| Titanium dioxide E171 | 12.000 | 0.89 |
| Gum arabic | 15.000 | 1.11 |
| **Sodium fluoride** | 1.105 | 0.08 |
| **Sodium bicarbonate** | 5.500 | 0.41 |
| **Phaseolamin** | 2.000 | 0.15 |
| Carnauba wax | 5.000 | 0.37 |
| E320 | 5.000 | 0.37 |
| | | |
| **TOTAL** | **1.350** | |

**EXAMPLE 5**

| **1.35 g ANTICARIES CHEWING GUM 2** | | |
|---|---|---|
| **Daily dosage: 1 dose after each meal** | | |
| **INGREDIENT** | **AMOUNT mg** | **%** |
| Sorbitol | 600.000 | 44.44 |
| Isomalt | 300.000 | 22.22 |
| Xylitol | 200.000 | 14.81 |
| Maltitol syrup | 94.395 | 6.99 |
| Gum base | 70.000 | 5.19 |
| Aspartame | 12.000 | 0.89 |
| Acesulfame K | 12.000 | 0.89 |
| Flavour | 12.000 | 0.89 |
| Titanium dioxide E171 | 12.000 | 0.89 |
| Gum arabic | 15.000 | 1.11 |
| **Sodium fluoride** | 1.105 | 0.08 |
| **Sodium bicarbonate** | 5.500 | 0.41 |
| ***Krameria triandra*** | 4.000 | 0.30 |
| **Phaseolamin** | 2.000 | 0.15 |
| Carnauba wax | 5.000 | 0.37 |
| E320 | 5.000 | 0.37 |
| | | |
| **TOTAL** | **1.350** | |

**EXAMPLE 6**

| **ANTICARIES TOOTHPASTE 1** | | |
|---|---|---|
| **750 ml TUBE** | | |
| **INGREDIENT** | **AMOUNT g** | **%** |
| **Calcium carbonate** | 300.000 | 40.00 |
| Water | 270.000 | 36.00 |
| Sorbitol | 89.250 | 11.90 |
| Hydrated silica | 20.000 | 2.67 |
| Sodium Lauryl Sulfate | 20.000 | 2.67 |
| **Sodium Monofluorophosphate** | 10.000 | 1.33 |
| Flavour | 8.000 | 1.07 |
| Cellulose Gum | 7.000 | 0.93 |
| Trisodium phoshate | 5.000 | 0.67 |
| Monosodium Phoshate | 5.000 | 0.67 |
| Sodium Saccharin | 5.000 | 0.67 |
| **Phaseolamin** | 3.750 | 0.50 |
| **Chlorhexidine** | 2.000 | 0.27 |
| CI74260 | 5.000 | 0.67 |
| | | |
| **TOTAL** | **750** | |

**EXAMPLE 7**

| **ANTICARIES TOOTHPASTE 2** | | |
|---|---|---|
| **750 ml TUBE** | | |
| **INGREDIENT** | **AMOUNT g** | **%** |
| **Calcium carbonate** | 300.000 | 40.00 |
| Water | 270.000 | 36.00 |
| Sorbitol | 85.250 | 11.37 |
| Hydrated silica | 20.000 | 2.67 |
| Sodium Lauryl Sulfate | 20.000 | 2.67 |
| **Sodium Monofluorophosphate** | 10.000 | 1.33 |
| Flavour | 8.000 | 1.07 |
| Cellulose Gum | 7.000 | 0.93 |
| **18β-glycyrrhizinic acid** | 6.000 | 0.80 |
| Trisodium phoshate | 5.000 | 0.67 |
| Monosodium Phoshate | 5.000 | 0.67 |
| Sodium Saccharin | 5.000 | 0.67 |
| **Phaseolamin** | 3.750 | 0.50 |
| CI74260 | 5.000 | 0.67 |
| | | |
| **TOTAL** | **750** | |

**EXAMPLE 8**

| **ANTICARIES TOOTHPASTE 1** | | |
|---|---|---|
| **750 ml TUBE** | | |
| **INGREDIENT** | **AMOUNT g** | **%** |
| **Calcium carbonate** | 300.000 | 40.00 |
| Water | 270.000 | 36.00 |
| Sorbitol | 88.250 | 11.77 |
| Hydrated silica | 20.000 | 2.67 |
| Sodium Lauryl Sulfate | 20.000 | 2.67 |
| **Sodium Monofluorophosphate** | 10.000 | 1.33 |
| Flavour | 8.000 | 1.07 |
| Cellulose Gum | 7.000 | 0.93 |
| Trisodium phoshate | 5.000 | 0.67 |
| Monosodium Phoshate | 5.000 | 0.67 |
| Sodium Saccharin | 5.000 | 0.67 |
| **Phaseolamin** | 3.750 | 0.50 |
| ***Krameria triandra*** | 3.000 | 0.40 |
| CI74260 | 5.000 | 0.67 |
| | | |
| **TOTAL** | **750** | |

## Claims

1. Phaseolamin for use as an anticaries agent.

2. Compositions comprising phaseolamin for use in the prevention of teeth carie.

3. The composition for use according to claim 2, in the form of mouth-soluble, slow-release tablets, candies or chewing gums.

4. The composition for use according to claim 2 in the semi-solid form.

5. The composition for use according to claim 4 in the form of gels or toothpastes.

6. The composition for use according to claim 2 in the liquid form.

7. The composition for use according to claim 6, in the form of mouth-washes.

8. Compositions for use according to any one of claims 1-7, further containing one or more of alkali carbonates or bicarbonates, sodium monofluorophosphate, inorganic fluorides, chlorhexidine, 18b-glycyrrhizinic acid, *Krameria triandra* extract, green tea.

## Patentansprüche

1. Phaseolamin zur Verwendung als ein Antikaries-Agens.

2. Zusammensetzungen umfassend Phaseolamin zur Verwendung in der Vorbeugung von Zahnkaries.

3. Die Zusammensetzung zur Verwendung gemäß Anspruch 2 in der Form von mundlöslichen Tabletten, Süßigkeiten oder Kaugummis mit langsamer Freisetzung.

4. Die Zusammensetzung zur Verwendung gemäß Anspruch 2 in der halbfesten Form.

5. Die Zusammensetzung zur Verwendung gemäß Anspruch 4 in der Form von Gelen oder Zahnpasten.

6. Die Zusammensetzung zur Verwendung gemäß Anspruch 2 in der flüssigen Form.

7. Die Zusammensetzung zur Verwendung gemäß Anspruch 6 in der Form von Mundspülungen.

8. Zusammensetzung zur Verwendung gemäß irgendeinem der Ansprüche 1-7, weiter enthaltend ein oder mehr Alkalicarbonate oder -bicarbonate, Natriummonofluorphosphat, anorganische Fluoride, Chlorhexidin, 18b-Glycyrrhizinsäure, *Krameria triandra-*Extrakt, grünen Tee.

## Revendications

1. Phaséolamine pour son utilisation à titre d'agent anticaries.

2. Compositions comprenant de la phaséolamine pour leur utilisation dans la prévention des caries dentaires.

3. Composition pour son utilisation selon la revendication 2, sous la forme de comprimés, de bonbons ou de chewing-gums à libération lente, solubles dans la bouche.

4. Composition pour son utilisation selon la revendication 2, sous une forme semi-solide.

5. Composition pour son utilisation selon la revendication 4, sous la forme de gels ou de dentifrices.

6. Composition pour son utilisation selon la revendication 2, sous une forme liquide.

7. Composition pour son utilisation selon la revendication 6, sous la forme de bains de bouche.

8. Compositions pour leur utilisation selon l'une quelconque des revendications 1 à 7, contenant en outre un ou plusieurs ingrédients choisis parmi des carbonates ou des bicarbonates de métaux alcalins, du monofluorophosphate de sodium, des fluorures inorganiques, de la chlorhexidine, de l'acide 18b-glycyrrhizinique, un extrait de *Krameria triandra,* du thé vert.
